(19) Europäisches Patentamt

European Patent Office

Office européen des brevets

(11) **EP 4 316 434 A1**

(12) **EUROPEAN PATENT APPLICATION**

(43) Date of publication:
**07.02.2024 Bulletin 2024/06**

(21) Application number: **23185995.0**

(22) Date of filing: **18.07.2023**

(51) International Patent Classification (IPC):
**A61F 9/008** (2006.01)

(52) Cooperative Patent Classification (CPC):
**A61F 9/008;** A61F 2009/00844; A61F 2009/00897

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **03.08.2022 CH 9262022**

(71) Applicant: **Ziemer Ophthalmic Systems AG 2562 Port (CH)**

(72) Inventors:
• **Steinlechner, Michael**
  **8006 Zürich (CH)**
• **Rathjen, Christian**
  **28197 Bremen (DE)**

(74) Representative: **Rentsch Partner AG Kirchenweg 8 Postfach 8034 Zürich (CH)**

(54) **OPHTHALMOLOGICAL LASER DEVICE HAVING A LASER SCANNER**

(57)    An ophthalmological laser device (1) is disclosed, the ophthalmological laser device (1) comprising: a base station (2) having: a treatment laser source (21) configured to generate a treatment laser beam, a scanner (22) arranged in a beam path of the treatment laser beam, wherein the scanner (22) comprises a deflecting element pivotable about two axes, wherein both axes lie in a virtual plane arranged substantially parallel to a deflecting plane of the deflecting element and the two axes are arranged orthogonal to each other; an application head (5); and an arm (4) arranged between the base station (2) and the application head (5).

Fig. 1

## Description

## FIELD OF THE DISCLOSURE

The present invention relates to an ophthalmological laser device having a laser scanner.

## BACKGROUND OF THE DISCLOSURE

Ophthalmological treatment devices, which use a laser for eye treatment, are known. The ophthalmological treatment device has a laser source, which produces a pulsed laser beam. Additionally, the wavelength of the laser light produced by the ophthalmological treatment device is dependent on the type of eye treatment and is typically in the ultraviolet (190nm to 230nm) or infrared (780nm to 1100nm) range.

The laser beam is typically produced by a laser source arranged in a base station. The laser beam is then guided along a beam path to an application head, where the laser beam is focused onto a patient's eye.

Downstream from the laser source, the laser beam is typically dynamically deflected by a scanner to generate the desired treatment pattern. Scanners are known which use, for example, a galvano actuator comprising one or more controllable mirrors (a so-called galvanoscanner). A disadvantage of galvanoscanners is their relatively high inertia and their relatively low angular resolution.

Additionally, scanner systems are known which comprise two movable deflecting elements, a first configured to deflect a laser beam in a lateral x-direction and a second configured to deflect a laser beam in a lateral y-direction. A disadvantage of such scanner systems is that they require two separate moving mirrors which increases the complexity of the scanner system as it is separated into two discrete components (the first and second deflecting elements are separate). This separation increases the size of the scanner systems. It also increases losses in laser power due to multiple deflections, each of them marginally reducing the laser power due to imperfect reflection. This arrangement also results in pincushion distortion of a laser pattern generated by the deflected laser beam, particularly for larger deflection angles.

## SUMMARY OF THE DISCLOSURE

It is an object of the invention and embodiments disclosed herein to provide an ophthalmological laser device having a scanner.

In particular, it is an object of the invention and embodiments disclosed herein to provide an ophthalmological laser device having a scanner which does not have at least some of the disadvantages of the prior art, in particular in that it has a low inertia, high angular resolution, and a compact design.

The present disclosure relates to an ophthalmological laser device. The ophthalmological laser device comprises a base station. The base station includes a treatment laser source configured to generate a treatment laser beam and a scanner arranged in a beam path of the treatment laser beam. The scanner is arranged downstream from the treatment laser source. The base station includes a control module connected to the treatment laser source and the scanner. The scanner comprises a deflecting element pivotable about a first axis and a second axis. Both axes lie in a virtual plane arranged substantially parallel to a deflecting plane of the deflecting element. The two axes are arranged substantially orthogonal to each other.

In an embodiment, the scanner comprises two or more actuators coupled to the deflecting element. The actuators are configured to cause, for example by linear extension, contraction, or by rotation, pivoting of the deflecting element about the first axis and/or the second axis. Thereby, the deflecting element is able to deflect an incident treatment laser beam in two dimensions, in particular extending in a lateral x-direction and a lateral y-direction with reference to a (quasi) two-dimensional x-y treatment plane orthogonal to a projection axis of the treatment laser beam in the eye of a patient.

In an embodiment, the scanner comprises two, three, or four piezoelectric actuators coupled to the deflecting element.

In an embodiment, the scanner comprises two, three, or four electromagnetic actuators coupled to the deflecting element.

In an embodiment, the scanner comprises two, three, or four electrostatic actuators coupled to the deflecting element.

In an embodiment, the scanner comprises a two-axis scan drive coupled to the deflecting element. The two-axis scan drive is configured to cause pivoting of the deflecting element about the first axis and/or the second axis.

In an embodiment, the ophthalmological laser device further comprises an application head and an arm arranged between the base station and the application head. The arm is configured to provide a beam path for the treatment laser beam.

In an embodiment, the arm is a rotatable arm, a telescopic arm, or an articulated arm. The arm can also comprise one or more rotatable, telescopic, or articulated parts or sections. The arm comprises one or more internal mirrors arranged in the beam path configured to guide the treatment laser beam from the base station to the application head.

In an embodiment, a length of the beam path in the arm is longer than a length of the beam path between the scanner and the arm and a length of the beam path in the application head. For example, the arm has a length of at least 50 cm, allowing for easy and flexible adjustment of the application head.

In an embodiment, the scanner is configured to have an angular resolution such that a minimum linear distance between treatment points (the minimum linear distance refers to a distance between laser spot centers) is less than 20 micrometers. In particular, the scanner is configured to have an angular resolution of less than 40 micro radians, preferably less than 20 micro radians. The angular resolution refers to a minimal configurable angular increment of the scanner. The angular resolution may apply to one or both axes, preferably both. The treatment points considered are arranged in a treatment surface (e.g., a treatment plane) orthogonal to a central axis of the beam path and lie on or in an eye of a patient.

In an embodiment, the scanner further comprises a scanner controller connected to the control module and the two or more actuators. The scanner comprises two or more strain gauges or other dilation sensitive sensors, each strain gauge connected to the scanner controller and attached to one of the actuators. The strain gauges are thereby configured to measure an elongation and/or a contraction of the actuators. The scanner controller is configured to receive, from the control module of the ophthalmological device, a scan signal. The scanner controller is configured to receive, from the two or more strain gauges, two or more strain gauge signals from the two or more strain gauges, respectively. Each strain gauge provides a strain gauge signal. The scanner controller is configured to generate a control signal using a scan signal received from the control module, the strain gauge signals, and closed loop control. The closed loop control is for example implemented as an algorithm which is executed by the scanner controller, taking as inputs the scan signal and the strain gauge signals, and providing as an output the control signal. The scanner controller is configured to transmit, to the actuators, the control signal for pivoting the deflecting element about the axes.

In an embodiment, the deflecting element is orientated with respect to the beam path of the treatment laser beam such that the treatment laser beam is deflected by 90°, when the deflecting element is in a zero position.

In an embodiment, the first axis and the second axis both form an angle of 45° to a virtual plane defined by the incident treatment laser beam and the deflected treatment laser beam, when the deflecting element is in a zero position.

In an embodiment, the ophthalmological laser device further comprises a scanner monitor. The scanner monitor includes a pilot light source and a scanner monitor sensor. The pilot light source is configured to generate a pilot light which is deflected by the deflecting element and then incident on the scanner monitor sensor. The scanner monitor is connected to the control module and configured to transmit to the control module a scanner monitor signal for monitoring the scanner.

In an embodiment, the control module is further configured to store a digital model of the scanner. The control module is configured to determine an actual dynamic motion of the scanner using the strain gauge signals and/or the scanner monitor signal. The control module is configured to generate a modelled dynamic motion of the scanner using the scan signal and the digital model. The control module is configured to determine whether the scanner is functioning properly by comparing the modelled dynamic motion with the actual dynamic motion.

## BRIEF DESCRIPTION OF THE DRAWINGS

The herein described disclosure will be more fully understood from the detailed description given herein below and the accompanying drawings, which should not be considered limiting to the invention described in the appended claims. The drawings in which:

Fig. 1    shows a block diagram illustrating schematically an ophthalmological laser device having a scanner;

Fig. 2    shows a perspective view illustrating schematically an ophthalmological laser device having a horizontally rotatable arm;

Fig. 3    shows a perspective view illustrating schematically an ophthalmological laser device having a vertically rotatable arm;

Fig. 4    shows a perspective view illustrating schematically an ophthalmological laser device having an articulated arm;

Fig. 5    shows a block diagram illustrating schematically a beam path of the treatment laser in the base station and the arm of an ophthalmological laser device;

Fig. 6    shows a block diagram illustrating schematically a scanner of an ophthalmological laser device;

Fig. 7    shows a perspective view illustrating schematically a scanner of an ophthalmological laser device;

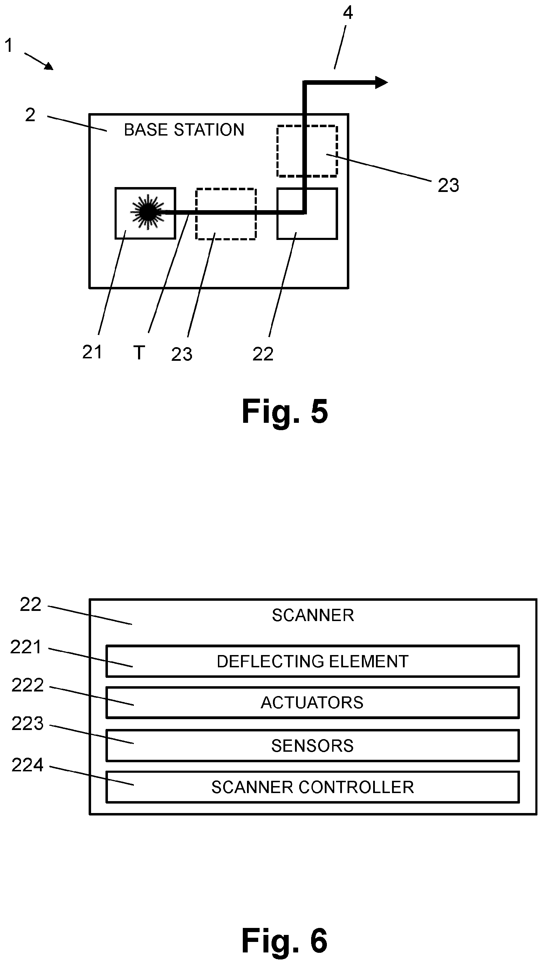

Figs. 6-11    show schematic illustrations of a deflecting element and a treatment laser beam deflecting off the deflecting element;

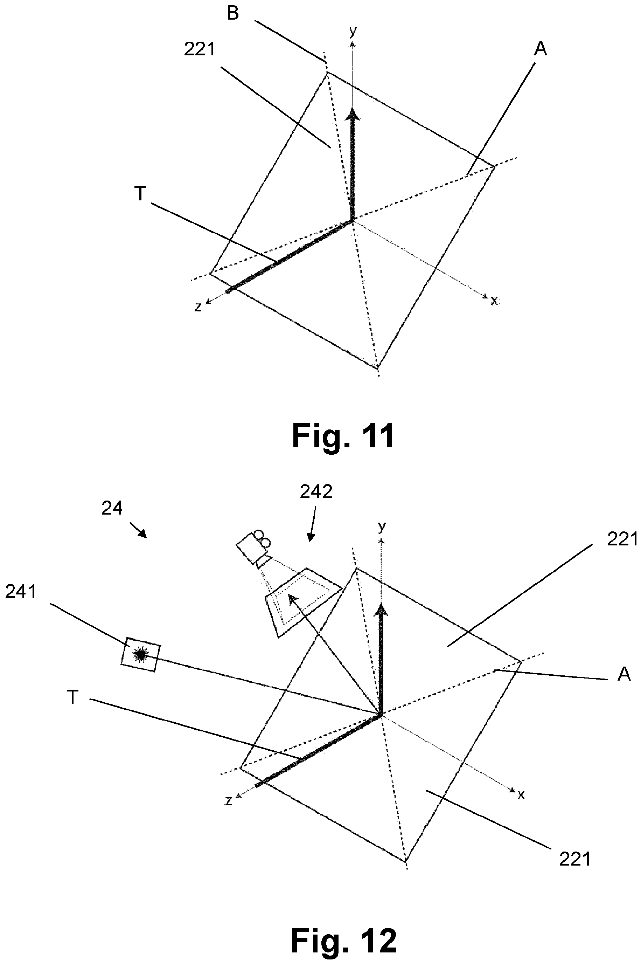

Fig. 12    shows a schematic illustration of a deflecting element and a scanner monitor including a pilot light source and a sensor;

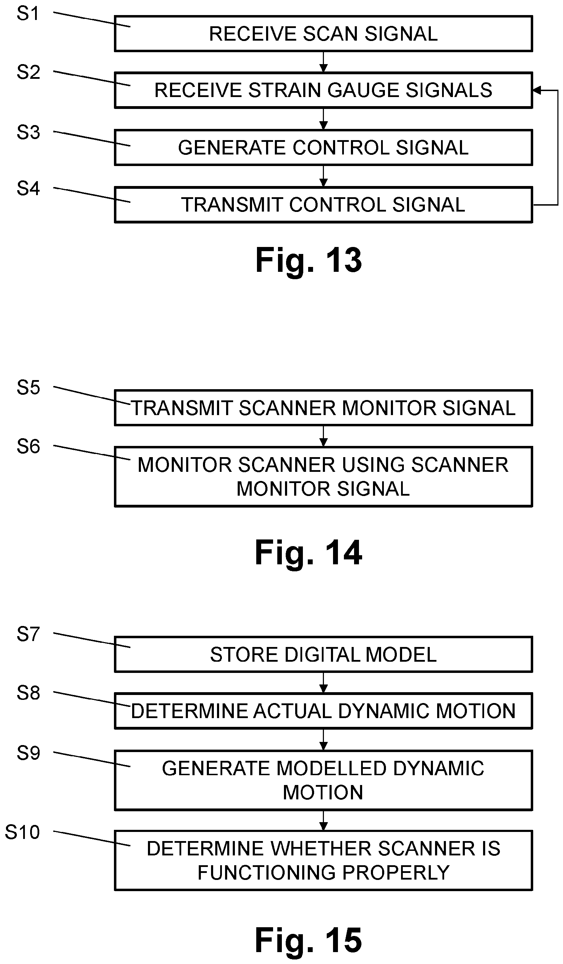

Fig. 13    shows a flow diagram illustrating an exemplary sequence of steps for generating a control signal;

Fig. 14    shows a flow diagram illustrating an exemplary sequence of steps for monitoring the scanner; and

Fig. 15    shows a flow diagram illustrating an exemplary sequence of steps for using a digital model of the scanner to determine whether the scanner is functioning properly.

**DESCRIPTION OF THE EMBODIMENTS**

Reference will now be made in detail to certain embodiments, examples of which are illustrated in the accompanying drawings, in which some, but not all features are shown. Indeed, embodiments disclosed herein may be embodied in many different forms and should not be construed as limited to the embodiments set forth herein; rather, these embodiments are provided so that this disclosure will satisfy applicable legal requirements. Whenever possible, like reference numbers will be used to refer to like components or parts.

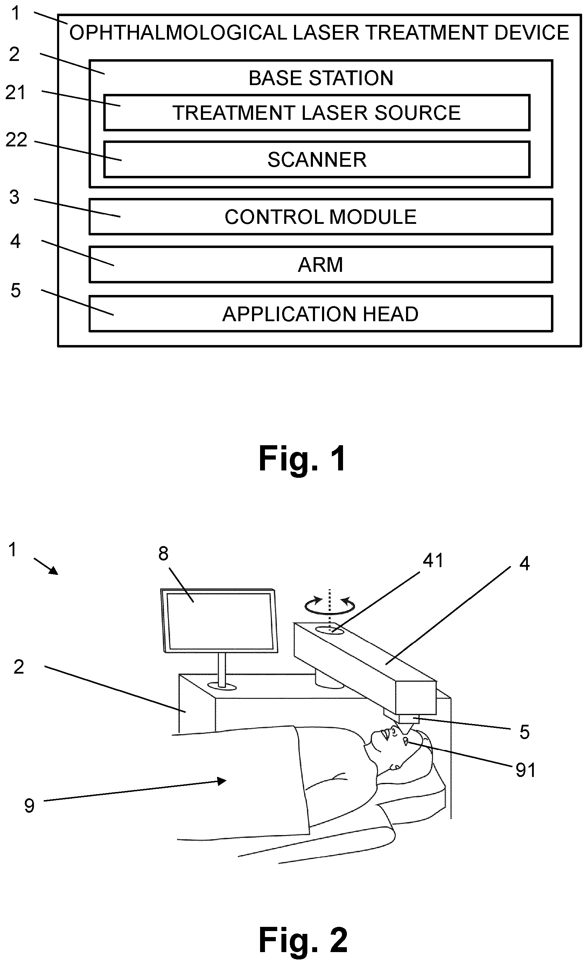

**Figure 1** shows a block diagram illustrating schematically an ophthalmological laser device 1 having a scanner 22. Figure 1 and also the remaining diagrams, in particular as included in Figures 2 - 7, schematically illustrate modules and/or elements of various embodiments of the ophthalmological laser device 1 and provide exemplary sequences or arrangement of modules and/or elements, including modules and/or elements in a beam path. The skilled person understands that at least some modules and/or elements shown in a particular Figure may be combined with modules and/or elements shown in another Figure.

The ophthalmological laser device 1 comprises a base station 2. The base station 2 is configured as a fixed or mobile apparatus. The ophthalmological laser device 1 has a treatment laser source 21 arranged in the base station 2 which generates a treatment laser beam T. The base station further includes, for example, a power supply and other auxiliary subsystems necessary for operation of the ophthalmological laser device 1.

The base station 2 may include a chassis. The chassis is designed to structurally support the weight of the components of the base station 2, such as the treatment laser source 21 and the scanner 22. The chassis may include one or more support members, e.g., struts, profiles, beams, and/or panels which may be connected to each other and/or to components of the base station 2.

The chassis is further designed to attach to the arm 4 of the ophthalmological laser treatment device 1, either directly or indirectly. The chassis may be designed to attach to a display 8 of the ophthalmological laser treatment device 1, either directly or indirectly.

The chassis may be configured to attach to and/or at least partially define a housing of the base station 2.

The base station 2 may include a housing. The housing may be attached to the chassis. The housing may consist of one or more panels. The housing at least partially encloses the base station 2 to shield and/or protect the internal components of the base station 2, for example from physical intrusion and/or dust collection.

The housing may include one or more locking doors for access to the internal components and/or one or more vents for air circulation inside the base station 2 (in particular for cooling purposes).

In an embodiment, the arm 4 is arranged on a top surface of the base station 2, the arm 4 being physically attached to the chassis and/or the housing of the base station 2. The arm 4 is configured to provide a beam path for the treatment laser beam T between the base station 2 and the application head 5.

The treatment laser source 21 is configured to, for example, generate an ultraviolet treatment laser beam T having a wavelength of between 190 nm and 230 nm. For example, the treatment laser source 21 comprises an excimer or a solid-state laser which produces such an ultraviolet treatment laser beam T. The excimer laser uses a combination of a noble gas and a reactive gas under high pressure and electrical stimulation to generate the treatment laser beam T. In particular, an excimer laser using argon as the noble gas and fluoride as the reaction gas may be used as the treatment laser source 21.

In another example, the treatment laser source 21 is configured to generate an infrared treatment laser beam T having a wavelength of between 780 nm and 1100 nm. For example, the treatment laser source 21 comprises a solid-state laser, such as a Ti:Sa or Yterbium laser. The treatment laser source 21 will not be described in further detail, however the skilled person is aware that the treatment laser source 21 can comprise, for example, a gain medium, a laser resonator, a laser pump, a pulse generating element, cavity mirrors, coupling mirrors, wavelength tuners, and/or a frequency converter (including one or more non-linear optical crystals) or fiber optical elements.

In an embodiment, the treatment laser beam T is a pulsed laser beam.

In an embodiment, the treatment laser source 21 is configured to generate femtosecond laser pulses, which have pulse widths of typically from 10 fs to 1000 fs (1 fs = $10^{-15}$ s).

The base station 2 includes a scanner 22 which is configured to steer the treatment laser beam T delivered by the treatment laser source 21 onto treatment points on a treatment pattern (comprising a laser trajectory). The scanner 22 is described in more detail with reference to Figures 6 to 12.

The ophthalmological laser device 1 comprises an application head 5. The application head 5 is designed to guide the treatment laser beam T into or onto the eye 91 of a patient 9 (as shown, for example, in Figures 2 to 4). The application head 5, for this purpose, can comprise focusing optics configured to focus the treatment laser beam T onto one or more treatment points inside or on the eye tissue, in particular the cornea for a pointwise tissue disruption or ablation. The focusing optics comprise a lens system having one or more optical lenses or mirrors. Depending on the embodiment, the focusing optics comprise one or more movable or deformable lenses and/or a drive for moving the entire focusing optics in order to set and adjust the focal depth, or the treatment height, in the projection direction along the projection axis.

In an embodiment, the application head 5 comprises a patient interface. The application head 5 is preferably fixed onto the eye 91 by means of the patient interface, for example using suction.

Alternatively, the application head 5 does not have a patient interface for direct contact with the eye 91, but the application head 5 and the eye 91 of the patient 9 are separated by an air gap of several centimeters, for example.

Depending on the embodiment, the ophthalmological laser device 1 (in particular, the application head 5) further comprises an eye tracker configured to track a position and/or orientation of the eye 91 of the patient 9.

The ophthalmological laser device 1 comprises an arm 4 arranged between the base station 2 and the application head 5. The arm 4 is configured to provide a beam path for the treatment laser beam T, such that the treatment laser beam T travels along the inside of the arm 4 from the base station 2 to the application head 5. In an embodiment, the arm 4 comprises one or more joints 41 (as shown in Figures 2-4) such that the application head 4 is movable and/or rotatable with respect to the base station 2. Each rotatable joint 41 comprises a mirror arranged in the beam path to reflect the treatment laser beam T along the arm 4.

In an embodiment, the ophthalmological laser device 1 comprises transmission optics arranged in the arm 4 and/or in the application head 5. The transmission optics are designed to work in conjunction with the scanner 22 to adapt the angular excursion of the ophthalmological laser device 1 to a treatment area and optionally to adjust the beam size. The transmission optics in particular can be an afocal system or a system with conjugated images planes. The transmission optics comprise one or more lenses and/or mirrors and may further include a drive for adjusting a focus and/or a reduction of image size.

Because the treatment laser beam T is reflected by each mirror, for example the mirrors in the arms 4, the treatment pattern generated by the scanner 22 is reflected and/or rotated according to the angles between the beam path and the mirror.

The ophthalmological laser device 1 is controlled by the control module 3, as described below in more detail, by controlling the treatment laser source 21 and the scanner 22, as well as by controlling additional modules of the ophthalmological laser device 1 arranged in the beam path of the treatment laser beam T, as described with reference to Figure 5 in more detail.

The control module 3 is configured to transmit, to the treatment laser source 21 and the scanner 22, control signals. The control signals include a laser control signal configured to control the treatment laser source 21 and a scan signal configured to control the scanner 22. The control signals can further comprise additional signals for additional modules of the ophthalmological laser device 1. The control signals are generated according to a treatment model which is designed for treating the eye 91 of a patient 9 using the ophthalmological laser device 1. The control signals are further generated according to operating specifications, operating parameters, monitoring routines and/or safety routines of the ophthalmological laser device 1. Additionally, the control module 3 can be configured to receive, from the treatment laser source 21 and the scanner 22 (optionally also from the additional modules), feedback signals. The feedback signals include, for example, a measurement signal from the treatment laser source 21 indicating a laser power, as well as scanner feedback signals from the scanner 22, indicating, for example, a position, orientation, and/or motion of the scanner 22 (including its components) as described below in more detail.

In an embodiment, the control module 3 is configured to check proper functioning of the scanner 22. The control module 3 checks proper functioning using the control signals (in particular the scan signal) and the feedback signal (in particular, the scanner feedback signal). Thereby, the following parameters of the scanner 22 may be assessed by the control module 3: a zero position of the scanner 22, a maximum deflection angle in one or more axes, a minimum angular deflection step size (i.e. minimal increment of deflection) in one or more axes), a flatness of the deflecting element 221, a distortion of the deflecting element 221, a dynamic response of the scanner 22, or other properties of the scanner 22.

The control module 3 is configured to control the ophthalmological laser device 1. The control module 3 is preferably arranged in the base station 2. The control module 3 embodies a programmable device and comprises, for example, one or more processors, and one or more memory modules having stored thereon program code, data, as well as programmed software modules for controlling the processors, and/or other programmable circuits or logic units included in the control module 3, such as ASICs (Application-Specific Integrated Circuits), GPUs (graphics processing units), and/orTPUs (tensor processing units). The memory modules comprise volatile and/or non-volatile storage media, for example random access memory and/or flash memory, respectively. The control module 3 is connected to other components and modules of the ophthalmological laser device 1 as disclosed herein, in particular the treatment laser source 21 and the scanner 22. The connection is a wired and/or wireless connection configured to exchange control signals and/or feedback signals.

The control module 3, depending on the embodiment, further comprises a communication interface. The communication interface is configured for data communication with one or more external devices. Preferably, the communication interface comprises a network communications interface, for example an Ethernet interface, a WLAN interface, and/or a wireless radio network interface for wireless and/or wired data communication using one or more networks, comprising, for example, a local network such as a LAN (local area network), and/or the Internet.

The control module 3 performs one or more steps and/or functions as described herein, for example according to the program code stored in the one or more memory modules. Additionally, or alternatively, the program code can be wholly or partially stored in one or more auxiliary processing devices, for example a computer. The skilled person is aware that at least some of the steps and/or functions described herein as being performed on the processor of the ophthalmological laser device 1 may be performed on one or more auxiliary processing devices connected to the ophthalmological laser device 1 using the communication interface. The auxiliary processing devices can be co-located with the ophthalmological laser device 1 or located remotely, for example on a remote server computer.

The skilled person is also aware that least some of the data associated with the program code (application data) or data associated with a particular patient (patient data) and described as being stored in the memory of the ophthalmological laser device 1 may be stored on one or more auxiliary storage devices connected to the ophthalmological laser device 1 using the communication interface.

The control module 3 stores, in the one or more memory modules, a treatment model. The treatment model is designed for treating the eye 91 of a patient 9 using the ophthalmological laser device 1. In particular, the treatment model defines a number of treatment points or treatment curves onto which the treatment laser beam T is directed.

In an embodiment, the control module 3 is configured to store, in the one or more memory modules, a digital model of the scanner 22. The digital model of the scanner 22 is a digital representation of the characteristics of the scanner 22, including structural characteristics and functional characteristics. The function of the digital model is explained in more detail below with reference to Figure 15.

Additionally, or alternatively, the control module 3 is configured to store one or more pre-determined thresholds related to properties of the ophthalmological treatment device 1, in particular the scanner 22. The pre-determined thresholds indicate, for example, normal or allowable limits and/or ranges of properties of the scanner 22. As explained below in more detail, the control module 3 is configured to monitor the ophthalmological treatment device 1, in particular the scanner 22, to ensure proper functioning, for example by monitoring whether the properties are within the pre-determined thresholds, as discussed below with reference to Figures 14 and 15.

The ophthalmological laser device 1 optionally includes a user interface comprising, for example, one or more user input devices, such as a keyboard, and one or more output devices, such as a display 8 (as shown in Figures 2-4). The user interface is configured to receive user inputs from an eye treatment professional, in particular based on, or in response to, information displayed to the eye treatment professional using the one or more output devices.

Figures 2 to 4 show three different embodiments of the ophthalmological laser device 1, each having a different embodiment of the arm 4. Other embodiments are possible, and depending on the configuration of the base station 2, in particular whether the base station 2 is itself height-adjustable or not, certain aspects of at least some of the three embodiments described below can be modified. Specifically, certain joints 41 for adjusting a vertical height of the application head 5 are superfluous, depending on the embodiment, and may be omitted.

In Figure 2, the arm is rotatable about the joint 51, such that the arm 5 can swing horizontally over a reclining patient 9 such that the application head 6 is moved into position above the eye 91 of the patient 9.

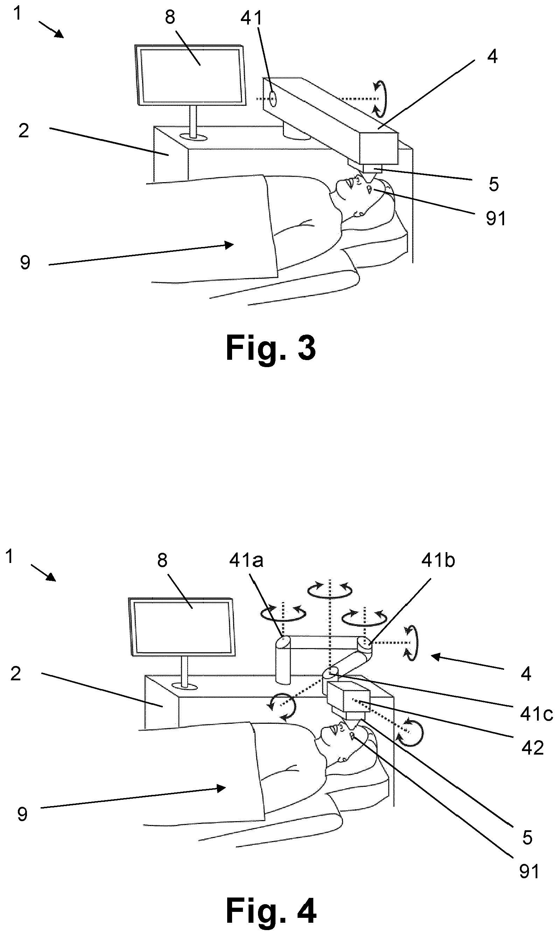

In Figure 3, the arm 5 is rotatable about the joint 41 such that the arm 5 can swing vertically over a reclining patient 9 such that the application head 5 is moved into position above the eye 91 of the patient 9.

In Figure 4, the arm 4 is an articulated arm 4 rotatable about the joints 41a, 41b, 41c such that the application head 5 can be flexibly moved into position above the eye 91 of the patient 9. Each joint 41a, 41b, 41c enables one or more rotations, for example the joint 41b allows both a rotation in the vertical as well as the horizontal plane. Additionally, it can be seen that the application head 5 has a joint 42 about which the application head 5 can be rotated.

The arm 4 as shown in Figures 2-4 preferably has a length of 50 cm or greater. This allows for flexible placement of the patient with respect to the base station 2. In particular, the embodiment shown in Figure 4 with an articulated arm 4 allows for the patient bed to be oriented flexibly with respect to the base station 2 and allows for simple accommodation of patients 9 of various heights and proportions.

The beam path along which the treatment laser beam T travels in the ophthalmological laser device 1 may be divided into several sections. A first section of the beam path is inside the base station 2, between the treatment laser source 21 and the scanner 22. A second section of the beam path is inside the base station 2, between the scanner 22 and the arm 5. A third section of the beam path is inside the arm 4. In a fourth section, the beam path is inside the application head 5. A length of the beam path in the third section (i.e. in the arm 4) is typically greater than a sum of a length of the beam path in the second section and fourth section.

Arranging the scanner 22 in the base station 2 has the advantage that the application head 5 remains compact and light-weight, however a drawback that must be overcome is that the greater distance between the scanner 22 and the application head 5 (and ultimately, the eye 91 of the patient 9) places greater demands on the capabilities of the scanner 22. At least some of the drawbacks are overcome by the scanner 22 disclosed herein.

**Figure 5** schematically shows the base station 2 and the arm 4, with the treatment laser beam T traveling along the beam path. The treatment laser beam T is produced by the treatment laser source 21 and is deflected by the scanner 22. By default (i.e., in the zero position), the scanner 22 deflects the treatment laser beam by 90°. The treatment laser beam T then exits the base station 2 and enters the arm 4, where it is guided towards the application head 5 (not shown).

Depending on the embodiment, one or more additional modules 23 are arranged in the beam path inside the base station 2. The one or more additional modules 23 are arranged downstream from the treatment laser source 21, upstream and/or downstream from the scanner 22. For example, some additional modules 23 may be arranged between the treatment laser source 21 and the scanner, and some additional modules 23 may be arranged between the scanner 22 and the arm 4.

The additional modules 23 include a laser attenuator, a beam shaper, a divergence modulator, a beam expander, and a shutter.

The laser attenuator is configured to attenuate the treatment laser beam T,

The beam shaper is configured to control the laser beam profile, in particular to redistribute the irradiance and/or phase of the treatment laser beam T to attain a desired laser beam profile that is maintained along the propagation distance, in particular the propagation distance from the beam shaper to the eye 91.

The divergence modulator is configured to modulate the focal depth and thereby the treatment height, in the projection direction along a projection axis.

The beam expander is configured to alter a diameter of the treatment laser beam T.

The shutter is arranged in the beam path and configured to stop the treatment laser beam T if an appropriate shutter signal is received.

Depending on the embodiment, one or more of the additional modules 23 are arranged in the arm 4 and/or in the application head 5.

As shown, the scanner 22 deflects the treatment laser beam T by 90° when in a zero position. The zero position is a default position of the scanner 22, i.e. a position the scanner 22 has in a default state. The zero position can include a zero position offset due to calibration.

In the embodiment shown in **Figure 6,** the scanner 22 comprises a deflecting element 221, one or more actuators 222, one or more scanner sensors 223, and a scanner controller 224. The scanner 22 has a scanner body to which the parts of the scanner 22 (i.e. the deflecting element 221, the one or more actuators 222, the one or more scanner sensors 223, and the scanner controller 224) are coupled, i.e. directly or indirectly connected. The connection includes a mechanical/structural part, but may also, additionally or alternatively, include an electrical connection for power and/or data transmission, depending on the nature of the part.

The deflecting element 221 is an element of the scanner 22 configured to deflect the treatment laser beam T. For example, the deflecting element 221 is a substantially flat surface which reflects the treatment laser beam T (more specifically, reflects a large proportion of the treatment laser beam T). The deflecting element 221 is, for example, implemented using a mirror, preferably a mirror having a low weight and/or rotational inertia, for example a beryllium mirror.

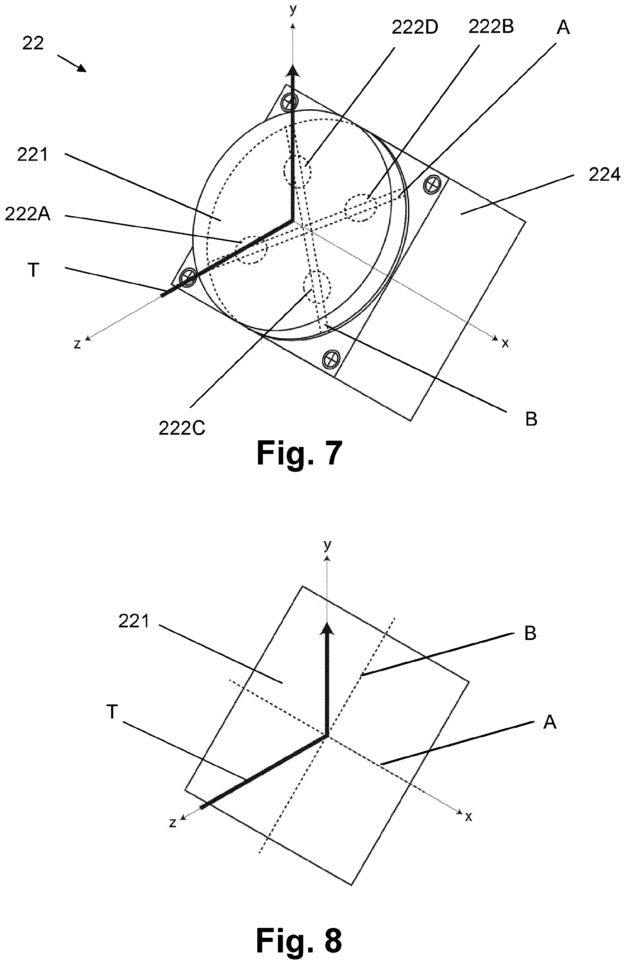

The deflecting element 221 is pivotable about a first axis A and a second axis B. The two axes A, B are arranged substantially orthogonal to each other, such that the incident treatment laser beam T is deflectable in two lateral dimensions (see Figures 7 to 12 for additional details). The deflecting element 221 is designed such that the two axes A, B are either coincident with a (deflecting) plane of the deflecting element 221. Alternatively, the two axes A, B lie in front of or behind the deflecting plane. In any case, both axes A, B lie in a virtual plane which is substantially parallel to the deflecting plane.

Depending on the embodiment, the pivoting of the deflecting element 221 is enabled by one or more actuators 222 directly. Alternatively or additionally, the deflecting element 221 pivots about one or more pivoting elements, including, for example, an axle and bearing, a ball and socket joint, a flexure bearing or other compliant mechanism or coupling. The pivots are passive elements which move in response to externally applied forces, in particular forces applied by the actuators 222. The pivots constrain the motion of the deflecting element 221.

In an embodiment, the deflecting element 221 has, for each of the two axes A, B, one or more pivots which pivotably couple the deflecting element 221 to a body of the scanner 22. Preferably, the pivots for the axes A, B, are nested (i.e. the pivots which enable the deflecting element 221 to pivot about the first axis A are coupled to the deflecting element 221 and to a support element, while the pivots which enable the deflecting element 221 to pivot about the second axis B are coupled to the support element and to the body of the scanner 22).

The one or more actuators 222 are coupled to the deflecting element 221, i.e. directly or indirectly connected to the deflecting element 221. The actuators 222 are configured to cause the deflecting element 221 to pivot about the axes A, B. For example, one of the actuators 222 is configured to pivot the deflecting element 221 about the first axis A, while another of the actuators 222 is configured to pivot the deflecting element 221 about the second axis B.

Depending on the type of actuator(s) 222 used, the pivoting can be effected by rotation of the actuator(s) 222 and/or extension (and/or contraction) of the actuator(s) 222, in particular linear extension.

In an embodiment, the scanner 22, in particular the actuator 222, comprises a two-axis scan drive configured to pivot the deflecting element 221 about the axes A, B.

In an embodiment, the actuators 222 are piezo-electric actuators coupled to the deflecting element 221 and configured to pivot the deflecting element 221 by linear extension. The actuators 222 may comprise two, three, or four piezo-electric actuators. Alternatively or additionally, the actuators 222 may comprise two, three, or four electromagnetic actuators. Alternatively or additionally, the actuators 222 may comprise two, three, or four electrostatic actuators.

In an embodiment, the actuators 222 comprise one or more bimorph actuators. The bimorph actuators can also enable pivoting of the deflecting element 221, such that separate pivot(s) are not required.

The scanner 22 is configured to allow for fast and minute changes in the orientation of the deflecting element 22 about the axes A, B. In particular, the scanner is configured to have an angular resolution sufficiently high such that a minimum linear distance between treatment points in the eye 91 of the patient 9 is less than 20 micrometers. The minimum linear distance refers to a distance between spot centers of the treatment laser beam T. The treatment points in this case are arranged orthogonal to a projection axis of the beam path of the treatment beam T. The high angular resolution is achieved by using actuators 222 with a small maximum relative displacement (e.g., piezo-electric actuators allow for a relative linear displacement on the order of 0.1%), arranging the actuators 222 at a sufficient distance from their pivot axis A, B, respectively, and controlling the actuators 222 with a control signal of sufficient resolution such that small incremental changes are possible. Fast changes in the orientation of the deflecting element 221 are enabled by designing the deflecting element 221 to have a low rotational inertia. For example, the deflecting element comprises a beryllium surface to achieve a low weight.

The scanner 22 comprises one or more scanner sensors 223. The sensor(s) 223 are configured to measure a state of the scanner 22, allowing for monitoring of the scanner 22.

The scanner sensors 223 are coupled to the deflecting element 221, the actuators 222, and/or the pivots of the scanner 22 and configured to measure or detect a position of the scanner 22, for example by directly measuring a position or movement of the deflecting element 221 or a position of the pivots, or by indirectly measuring a position or movement of the actuators 222.

In an embodiment, the scanner sensors 223 are implemented as strain gauges coupled to the actuators 222. Each actuator 222 is coupled to one or more strain gauges. The strain gauges are electrically connected to the scanner 22, in particular the scanner controller 223 described below in more detail, and provide, upon having a measurement signal passed through them, strain gauge signals which are indicative of a linear extension and/or contraction of the strain gauge and therefore also of the actuator 222 to which they are coupled.

The scanner 22 comprises a scanner controller 224. The scanner controller 224 is implemented, for example, as an integrated circuit including a microprocessor. The scanner controller 224 is connected to the actuator(s) 222 and the sensor(s) 223. The scanner 22 includes, or is connected to, power electronics for powering the scanner 22. The scanner controller 224 is configured to control the scanner 22, in particular to cause a movement of the deflecting element 221 by transmitting control signals to the actuators 222. The scanner controller 224 is configured to receive sensor signals from the scanner sensors 223 (e.g., strain gauge signals from the strain gauges).

The scanner controller 224 is configured to control the scanner 22 according to a scan signal received from the control module 3. For example, the scan signal is indicative of a particular position or movement of the scanner 22 (in particular, the deflecting element 221), and the scanner controller 224 positions or moves the scanner 22 accordingly (in particular, by controlling the actuators 222). As described with reference to Figure 13 below in more detail, the scanner control 224 is, in an embodiment, configured to implement closed loop control.

**Figure 7** shows a scanner 22 with a deflecting element 221. The deflecting element 221 has a circular shape with a flat surface and is designed to reflect the treatment laser beam T. As shown in the Figure, the treatment laser beam T arrives from the negative z-direction and strikes the surface of the deflecting element 221 in its center (i.e. the origin of the coordinate system used in the Figure). For purposes of illustration, the treatment laser beam T as shown in the Figure merely depicts a beam center of the treatment laser beam T - in reality the treatment laser beam T is wider than depicted. The treatment laser beam T is deflected by 90° by the deflecting element 221 and continues traveling in the positive y-direction. In the state as shown in the Figure, the deflecting element 221 is in a zero position. The zero position is a default position. The zero position is achieved, for example, with the actuators 222 in their default (i.e. unactuated) position. The zero position can also be subject to calibration. In the zero position, the treatment laser beam T is typically deflected by 90°.

By pivoting the deflecting element 221 about one or both of the axes A, B, the treatment laser beam T is deflected from the y-direction such that the vector of the treatment laser beam T has a vector component in the x-direction and/or the z-direction in the coordinates of the Figure. However, this translates to, for example, a deflection into the x and y direction in the (quasi) two-dimensional treatment plane of the surface of the eye 91.

In the embodiment shown, the axes A, B about which the deflecting element 221 pivots are arranged at 45° to the x-direction in the plane of the surface of the deflecting element 221. Further, the plane formed by the incident and deflected treatment laser beam T is orthogonal to the x-direction (i.e. both the incident treatment laser beam T and the deflected treatment laser beam T are orthogonal to the x-direction), and therefore the axes A, B both form an angle of 45° to the plane formed by the incident and deflected treatment laser beam T.

As explained with reference to **Figures 8-10** below in more detail, this ensures that the scanner 22 has, for both axes A, B, the same sensitivity in deflecting the treatment laser beam T.

The actuators 222 are arranged parallel to the axes A, B, respectively. In particular, a first set of two actuators 222A, 222B, is arranged behind the deflecting element 221 parallel to the first axis A and configured to pivot the deflecting element 221 about the second axis B. Analogously, a second set of two actuators 222C, 222D, is arranged behind the deflecting element 221 parallel to the second axis B and configured to pivot the deflecting element 221 about the first axis A.

The actuators 222 are configured to pivot the deflecting element 221 about the axes A, B by linear extension. In particular, a particular actuator 222A of the first set of actuators is configured to extend linearly to pivot the deflecting element about the second axis B in a first direction of rotation. During rotation in the first direction, the other actuator 222B of the first set of actuators 222A, 222B linearly contracts. The linear contraction may be passive, i.e. the other actuator 222B does not provide a contracting force but is contracted due to the force exerted by the linear expansion of the particular actuator 222A. For rotation in a second direction of rotation opposite to the first, the actuators 222A, 222B work in reverse fashion. Similar considerations apply for the second set of actuators 222C, 222D configured to pivot the deflecting element 221 about the first axis A.

The actuators 222 are arranged to be far from the axis A, B which they are configured to pivot (in particular, closer to an edge of the deflecting element 221 than to the axis A, B which they pivot), such that a given linear extension of the actuators 222 results in a small angular pivoting of the deflecting element 221 due to lever action.

The actuators 222 are preferably piezo-electric actuators 222 configured to extend linearly when an appropriate control signal is applied. The scanner sensors 223 (not shown) are configured to measure the linear extension of the actuators 222. Preferably, the scanner sensors 223 are strain gauges coupled to the actuators 222.

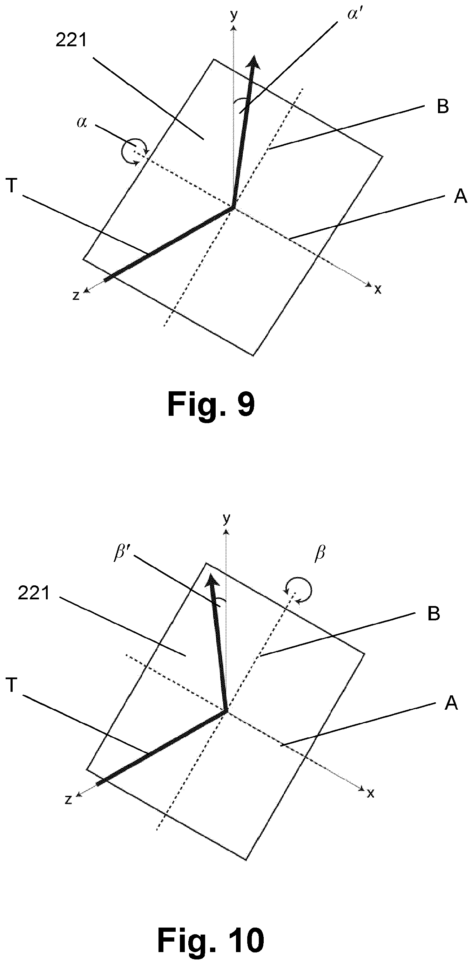

**Figure 8** shows the treatment laser beam T deflecting off the deflecting element 221 with the deflecting element 221 in a zero position. In the example as shown in the Figure, the deflecting element 221 is depicted as having a rectangular flat surface and, for illustrative purposes, the axis A is coincident with the x-direction and the axis B forms an angle of 45° with the y-direction and lies in the zy plane. As shown in **Figure 9**, in this configuration, a pivoting of the deflecting element 221 about the first axis A by an angle $\alpha$ results in a deflection of the treatment laser beam T by an angle $\alpha'$ in the negative z-direction in the yz plane, where $\alpha' = 2\alpha$ due to the law of reflection. As shown in **Figure 10**, in this configuration, a pivoting of the deflecting element 221 about the second axis B by an angle $\beta$ results in a deflection of the treatment laser beam T by an angle $\beta'$ in the negative x direction in the xy plane, wherein $\beta' = \sqrt{2}\beta$. This is due to the inclined orientation of the deflecting element 221 (in particular the second axis B) with respect to the deflected treatment laser beam T. The aforementioned relation is valid for small angles. Therefore, the scanner 22, in particular the deflecting element 221, has a reduced angular resolution for pivots about the second axis B as compared with pivots about the first axis A.

For example, if the scanner 22 is to scan a circle, then the deflected treatment laser beam T must circle the y-direction at a constant angle to the y-axis. To achieve such a circling with constant angle, the deflecting element 221 must, however, pivot more about the second axis B than the first axis A.

Therefore, it is advantageous to arrange the deflecting element 221 as shown in **Figures 7** and **11**, where both axes A, B form an angle of 45° to the plane formed by the incident treatment laser beam T and the reflected treatment laser beam T. Thereby, both axes A, B contribute equally to the deflection of the treatment laser beam T in that they are both equally sensitive to pivots about their respective axes A, B.

Reference numeral 24 in **Figure 12** refers to a scanner monitor 24. The scanner monitor 24 is included in the base station 2 in an embodiment. The scanner monitor 24 is connected to the control module 3 and/or the scanner controller 224 and is configured to monitor the scanner 22, in particular the deflecting element 221, by means of monitoring a pilot light reflected off the deflecting element 221. In this manner, the state of the deflecting element, including its position, motion, and/or surface condition is monitored.

In an embodiment, the scanner monitor 24 or parts thereof are integrated into the scanner 22. For example, the integration includes a structural integration such that the scanner monitor 24 is directly attached to the scanner 22, as opposed to attached to the base station 2 or other components. The integration may also include, for example, a functional integration with the scanner 22 such that the operation of the scanner monitor 24 is coordinated with the operation of the scanner 22.

The scanner monitor 24 includes a pilot light source 241 and a scanner monitor sensor 242.

The pilot light source 241 is configured to generate a pilot light which is reflected by the deflecting element 221 and received by the scanner monitor sensor 242. The pilot light includes, for example, a LED light and/or a laser. The pilot light source 241 is arranged in the base station 2. Preferably, the pilot light source 241 is arranged such that the pilot light is directly incident on the deflecting element 221, i.e. that there are no optical elements arranged between the pilot light source 241 and the deflecting element 211. Alternatively, optical elements (e.g., including a mirror and/or a lens) are arranged between the pilot light source 241 and the deflecting element 211.

In an embodiment, the pilot light source 241 is integrated into the scanner 22. In particular, the pilot light source 241 is structurally attached to the scanner 22. Thereby, the constant angle of incidence of the pilot light onto the deflecting element 221 is ensured. In particular, the pilot light source 241 is configured such that the pilot light is incident on the deflecting element 221 with an oblique angle, preferably an angle smaller than 45° from a surface normal of the deflecting element 221. The pilot light source 241 is typically directed at a center of the deflecting element 221, however other designs are also possible.

The scanner monitor sensor 242 is arranged such that the reflected pilot light is incident on the scanner monitor sensor 242. The scanner monitor sensor 242 has a sensing surface area configured such that the pilot light is incident on the sensing surface area, regardless of the position and/or movement of the deflecting element 221 of the scanner 22. To this end, the scanner monitor sensor 242 is typically arranged such that the pilot light strikes roughly the center of the sensing surface area when the deflecting element 221 is in the zero position.

In an embodiment, the scanner monitor sensor 242 is integrated into the scanner 22. In particular, the sensor is structurally attached to the scanner 22.

The scanner monitor sensor 242 comprises a photodetector array, for example a one-dimensional, two-dimensional, or three-dimensional array of photodetectors. The photodetector array is implemented, depending on the embodiment, as a CCD sensor or a CMOS sensor, for example. The photodetector array may comprise a movable shutter.

Additionally or alternatively, the scanner monitor sensor 242 comprises a photodiode, a position sensitive device, an optical power sensor, a microbolometer, and/or a pyroelectric detector.

The scanner monitor sensor 242 is configured, depending on the embodiment, to be sensitive to one or more wavelengths (this includes a wavelength band) of light. The wavelengths to which the scanner monitor sensor 242 may be sensitive are not limited to visible wavelengths, but are additionally or alternatively also sensitive to light beyond the visible range, for example ultraviolet light and/or infrared light. The wavelength(s) to which the sensor 24 is sensitive depends on the wavelength of light produced by the pilot light source 241 and optionally, the wavelength of the treatment laser beam T.

In an embodiment, scanner monitor sensor 242 comprises a matte screen onto which the reflected pilot light is incident. The scanner monitor sensor 242, specifically the photo-sensitive components of the sensor which transduce the optical signal to an electrical signal, thereby indirectly monitors the pilot light.

The scanner monitor sensor 242 is configured to generate a scanner monitor signal depending on the properties incident pilot light. The scanner monitor signal is transmitted to the control module 3 and/or the scanner controller 224. The properties of the incident pilot light include its beam characteristics, light position, and/or light motion. The beam characteristics comprise, for example, a light intensity and a light profile. The light position relates to the two-dimensional position of the pilot light on the scanner monitor sensor 242 and is indicate of, for example, the zero position, as well as the current orientation of the deflecting element 221. The light motion relates to a dynamic motion of the pilot light on the scanner monitor sensor 242 and is indicative of, for example, an angular velocity of the deflecting element 221.

The aforementioned properties of the incident pilot light are useful for identifying, for example, a contaminated deflecting element 221, a defective deflecting element 221, or otherwise improper functioning of the scanner 22. Described below in more detail with reference to Figure 14 are a number of steps performed by the scanner monitor 24 to monitor the scanner 22.

**Figure 13** illustrates a sequence of steps performed by the scanner controller 224 in an embodiment.

In a step S1, the scanner controller 224 is configured to receive the scan signal from the control module 3, the scan signal being generated by the control module 3 according to the treatment model.

In a step S2, the scanner controller 224 is configured to receive sensor signals from the scanner sensors 223, in particular strain gauge signals received from strain gauges, which sensor signals provide feedback regarding the position or movement of the deflecting element 221, either directly, or via a position or motion of the actuators 222.

In a step S3, the scanner controller 224 is configured to generate a control signal for the actuators 222, the control signal being generated using the scan signal and the sensor signals.

In a step S4, the scanner controller is configured to transmit the control signal to the actuators 222.

The scanner controller 224 is configured to monitor the sensor signals by performing step S2 and then generating S3 and transmitting S4 an adjusted control signal until the sensor signals indicate that the scanner 22, in particular the deflecting element 221, has reached the target position or target motion as defined by the scan signal.

Thereby, the scanner controller 224 performs closed loop control by monitoring an actual current position or movement of the scanner 22 as indicated by the sensor signals and comparing this to a target position or movement as indicated by the scan signal to generate an appropriate control signal for the actuators 222. The closed loop control is implemented in the scanner controller 224 using appropriate circuitry and/or stored instructions (e.g., algorithms or routines). For example, the closed loop control comprises a PID controller implemented in the scanner controller 224.

**Figure 14** illustrates a sequence of steps performed in the ophthalmological treatment device 1, in particular by the scanner monitor 24. The scanner monitor 24, in particular the scanner monitor sensor 242, is configured for continuous monitoring of the scanner 22. Preferably, the scanner monitor 24 is configured for real-time monitoring.

In a step S5, the scanner monitor 24 is configured to continuously (preferably in real-time) transmit the scanner monitor signal to the control module 3 and/or the scanner controller 224.

In a step S6, the control module 3 is configured to monitor the scanner 24 using the scanner monitor signal. The control module 3 includes, for example, one or more pre-determined thresholds related to the properties of the scanner 22, the deflecting element 221, and/or the pilot light as detected by the scanner monitor sensor 242. The control module 3 is configured to check, preferably on a continual basis, more preferably on a real-time basis, whether the scanner 22 is operating within the one or more pre-determined thresholds using the scanner monitor signal. Depending on the embodiment, the control module 3 can further use the sensor signals from the scanner sensors 223.

**Figure 15** illustrates an exemplary sequence of steps performed in an embodiment where the ophthalmological laser device 1 comprises a digital model of the scanner 22. The digital model is a digital representation of the scanner 22 and includes, for example, a model of the physical behavior of the scanner 22 and the components thereof, including in particular the deflecting element 221.

The model is, for example, a parametrized model which includes equations or algorithms which govern the static and/or dynamic behavior of the scanner 22, which equations or algorithms are parameterized according to the parameters of the scanner 22. The parameters of the deflecting element 221 include an inertia, in particular a rotational inertia of the deflecting element 221. The model is designed to simulate the scanner 22. The parametrized model, during simulation, takes as an input one or more time-varying input variables, for example the scan signal, and generates one or more time-varying output variables, defining one or more positions and/or one or more angles of the deflecting element 221. Additional output variables may include dynamic values including linear velocities and/or angular velocities of the deflecting element 221.

The angular velocity of the deflecting element 221 is, for example, an output variable which depends on the scan signal input. The model accounts for the torque about the pivot produced by the actuators 222 and a rotational inertia of the deflecting element 221., In another example, the model is configured to directly output, or to enable the determination of, a spot settling time indicative of a characteristic time for the deflecting element 221 to settle on a determined position. The model accounts for the torque, the rotational inertia, as well as damping characteristics of the actuators, pivots, etc.

In another embodiment, the model is a black box type model which is not explicitly programmed with equations, algorithms, or parameters designed to reflect the physical behavior of the scanner 22. Rather, the model is fitted or trained to reflect the physical behavior of the scanner 22. For example, the model is trained, using machine learning, to model the scanner 22. During training, the model is provided with exemplary input variables and therewith associated exemplary output variables and iteratively adapts an internal set of parameters to approximate the physical behavior of the scanner 22. The model can include a neural network, for example.

In a step S7, the control module 3 is configured to store the digital model of the scanner 22 in the memory. The digital model is established based on the design and/or measurements of the scanner 22 and can include values established during calibration.

In a step S8, the control module 3 is configured to determine an actual dynamic motion of the scanner 22 using the sensor signals received from the scanner sensor 223 (in particular the strain gauge signals) and/or the scanner monitor signal received from the scanner monitor 24.

In a step S9, the control module 3 is configured to determine a modelled dynamic motion of the scanner 22 using the scan signal and the digital model. Thereby, the control module 3 is configured to simulate the scanner 22, in particular to predict a motion of the deflecting element 221, based on the scan signal provided to the scanner 22 and the digital model of the scanner 22.

In a step S10, The control module 3 is configured to compare the actual dynamic motion of the scanner 22, as determined from the measurements as provided by the sensor signals and/or the scanner monitor signal, with the modelled dynamic motion (i.e. the predicted motion). The control module 3 is configured to determine whether the scanner 22 is functioning properly using the comparison.

For example, the control module 3 is configured to determine whether the scanner 22 is functioning properly by determining a deviation of the actual dynamic motion from the modelled dynamic motion. In particular, the scanner 22 is determined to be functioning properly if the deviation is below one or more pre-determined thresholds. If the deviation is greater than or equal to the one or more pre-determined thresholds, the scanner 22 is deemed not to be functioning properly and, depending on the embodiment, the control module 3 may be configured to generate a warning signal, for example configured to be displayed prominently on the display 8 of the ophthalmological laser device 1. Additionally or alternatively, the control module 3 may be configured to shut down one or more modules or components of the ophthalmological laser device 1, in particular the treatment laser source 21 and/or the scanner 22.

In an embodiment, the scanner controller 224 may use the scanner monitor signal for closed loop control instead of, or in addition to, the sensor signals received from the scanner sensor 224 (e.g., the strain gauge signals).

In an embodiment, the scanner monitor sensor 242 further comprises one or more filters. The filters are configured to absorb, scatter, convert and/or reflect one or more wavelengths of light. Depending on the embodiment, the filters may further be configured to re-emit energy any absorbed energy in a specific wavelength region.

The filters comprise, for example, one or more color filters, one or more spectral filters, one or more neutral density filters, one or more band pass filters, one or more notch filters, one or more edge filters, one or more beam splitter filters, one or more dichroic filters, one or more color substrate filters, one or more excitation filters, and/or one or more emission filters. The filters can cover the entire scanner monitor sensor 242 or parts thereof, including, for example, individual areas (e.g., a Bayer color filter may be used where the scanner monitor sensor 242 is implemented as a photodetector array).

The scanner monitor sensor 242 comprises, in an embodiment, optical elements such as mirrors and/or lenses to focus and/or distribute the light signal across the photodetector array. For example, the optical elements include microlenses arranged in front of the photodetector array to increase the light signal detected at each photodetector.

The above-described embodiments of the disclosure are exemplary and the person skilled in the art knows that at least some of the components and/or steps described in the embodiments above may be rearranged, omitted, or introduced into other embodiments without deviating from the scope of the present disclosure.

**Claims**

1. An ophthalmological laser device (1), the ophthalmological laser device (1) comprising:

    a base station (2) having:

    a treatment laser source (21) configured to generate a treatment laser beam (T), a scanner (22) arranged in a beam path of the treatment laser beam (T) downstream from the treatment laser source (21), the scanner (22) comprises a deflecting element (221) pivotable about a first axis (A) and a second axis (B), wherein both axes (A, B) lie in a virtual plane arranged substantially parallel to a deflecting plane of the deflecting element (221) and the two axes (A, B) are arranged orthogonal to each other, and

    a control module (3) connected to the treatment laser source (21) and the scanner (22);

    an application head (5); and
    an arm (4) arranged between the base station (2) and the application head (5), wherein the arm (4) is configured to provide a beam path for the treatment laser beam (T).

2. The ophthalmological laser device (1) of claim 1, wherein the scanner (22) comprises two or more actuators (222) coupled to the deflecting element (221) and configured to cause, by linear extension, pivoting of the deflecting element (221) about at least one of the following: the first axis (A) or the second axis (B).

3. The ophthalmological laser device (1) of one of claims 1 or 2, wherein the scanner (22) comprises two, three, or four piezoelectric actuators (222) coupled to the deflecting element (221).

4. The ophthalmological laser device (1) of one of claims 1 or 2, wherein the scanner (22) comprises two, three, or four electromagnetic actuators (222) coupled to the deflecting element (221).

5. The ophthalmological laser device (1) of one of claims 1 or 2, wherein the scanner (22) comprises two, three, or four electrostatic actuators (222) coupled to the deflecting element (221).

6. The ophthalmological laser device (1) of one of claims 1 to 5, wherein the scanner (22) comprises a two-axis scan drive coupled to the deflecting element (221) and configured to cause pivoting of the deflecting element (221) about at least one of the following: the first axis (A) or the second axis (B).

7. The ophthalmological laser device (1) of one of claims 1 to 6, wherein the arm (4) is one or more of the following: a rotatable, telescopic, or articulated arm, comprising one or more internal mirrors arranged in the beam path.

8. The ophthalmological laser device (1) of one of claims 1 to 7, wherein a length of the beam path in the arm (4) is longer than a length of the beam path between the scanner (22) and the arm (4) and a length of the beam path in the application head (5).

9. The ophthalmological laser device (1) of one of claims 1 to 8, wherein the scanner (22) is configured to have an angular resolution such that a minimum linear distance between treatment points arranged orthogonal to a central axis of the beam path, where the treatment points are in an eye of a patient, is less than 20 micrometers.

10. The ophthalmological laser device (1) of one of claims 2 to 9, wherein the scanner (22) further comprises:

- a scanner controller (224) connected to the control module (3) and the two or more actuators (222), and
- two or more strain gauges, each strain gauge connected to the scanner controller (224) and attached to one of the actuators, wherein the scanner controller (224) is configured to:

- receive (S1), from the control module (3), a scan signal and receive (S2), from the two or more strain gauges, two or more strain gauge signals from the two or more strain gauges, respectively,
- generate (S3) a control signal using a scan signal received from the control module (3), the strain gauge signals, and closed loop control, and
- transmit (S4), to the actuators (222), the control signal for pivoting the deflecting element (221) about the axes (A, B).

11. The ophthalmological laser device (1) of one of claims 1 to 10, wherein the deflecting element (221) is orientated with respect to the beam path of the treatment laser beam (T) such that treatment laser beam (T) is deflected by 90°, when the deflecting element (221) is in a zero position.

12. The ophthalmological laser device of one of claims 1 to 11, wherein the first axis (A) and the second axis (B) both form an angle of 45° to a virtual plane defined by the incident treatment laser beam (T) and the deflected treatment laser beam (T), when the deflecting element (221) is in a zero position.

13. The ophthalmological laser device of one of claims 1 to 12, further comprising a scanner monitor (24) including a pilot light source (241) and a scanner monitor sensor (242), wherein the pilot light source (241) is configured to generate a pilot light which is deflected by the deflecting element (221) and incident on the scanner monitor sensor (242), wherein the scanner monitor (24) is connected to the control module (3) and configured to transmit to the control module (3) a scanner monitor signal for monitoring the scanner (22).

14. The ophthalmological laser device of one of claims 10 to 13, wherein the control module (3) is further configured to:

- store (S7) a digital model of the scanner (22),
- determine (S8) an actual dynamic motion of the scanner (22) using one or more of: the strain gauge signals or the scanner monitor signal,
- generate (S9) a modelled dynamic motion of the scanner (22) using the scan signal and the digital model, and
- determine (S10) whether the scanner (22) is functioning properly by comparing the modelled dynamic motion with the actual dynamic motion.

1

OPHTHALMOLOGICAL LASER TREATMENT DEVICE

2

BASE STATION

21

TREATMENT LASER SOURCE

22

SCANNER

3

CONTROL MODULE

4

ARM

5

APPLICATION HEAD

## Fig. 1

1

8

41

4

2

5

91

9

## Fig. 2

1

8    41                                    4

2                                                  5

                                                   91

9

# Fig. 3

1

8    41a          41b

2                                              4

                                               41c

                                               42

9                                              5

                                               91

# Fig. 4

1

4

2     BASE STATION                  23

21    T    23    22

## Fig. 5

22    SCANNER

221    DEFLECTING ELEMENT

222    ACTUATORS

223    SENSORS

224    SCANNER CONTROLLER

## Fig. 6

**Fig. 7**

**Fig. 8**

**Fig. 9**

**Fig. 10**

## Fig. 11

## Fig. 12

S1 — RECEIVE SCAN SIGNAL

S2 — RECEIVE STRAIN GAUGE SIGNALS

S3 — GENERATE CONTROL SIGNAL

S4 — TRANSMIT CONTROL SIGNAL

## Fig. 13

S5 — TRANSMIT SCANNER MONITOR SIGNAL

S6 — MONITOR SCANNER USING SCANNER MONITOR SIGNAL

## Fig. 14

S7 — STORE DIGITAL MODEL

S8 — DETERMINE ACTUAL DYNAMIC MOTION

S9 — GENERATE MODELLED DYNAMIC MOTION

S10 — DETERMINE WHETHER SCANNER IS FUNCTIONING PROPERLY

## Fig. 15

Europäisches
Patentamt

European
Patent Office

Office européen
des brevets

# EUROPEAN SEARCH REPORT

**Application Number**

**EP 23 18 5995**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/015553 A1 (ZACHARIAS JAIME [US]) 17 January 2008 (2008-01-17) | 1-13 | INV. A61F9/008 |
| A | * paragraphs [0065], [0066], [0074], [0077], [0083], [0089], [0090], [0099] – [0104], [0110] – [0112]; figures 2,4,6B,7B,7C,8A,8B,9,11 * | 14 | |
| A | US 4 538 608 A (L ESPERANCE JR FRANCIS A [US]) 3 September 1985 (1985-09-03) * column 3, line 39 – line 62 * | 1-14 | |
| A | WO 2021/155445 A1 (ELLEX MEDICAL PTY LTD [AU]) 12 August 2021 (2021-08-12) * page 9, line 5 – line 23; figure 4 * | 1-14 | |
| A | US 2012/029491 A1 (RATHJEN CHRISTIAN [DE]) 2 February 2012 (2012-02-02) * paragraph [0041] * | 1-14 | |
| A | US 2021/100688 A1 (BUSS THOMAS [DK] ET AL) 8 April 2021 (2021-04-08) * paragraph [0087] – paragraph [0090] * | 1-14 | TECHNICAL FIELDS SEARCHED (IPC) A61F |

The present search report has been drawn up for all claims

| Place of search | Date of completion of the search | Examiner |
|---|---|---|
| Munich | 12 September 2023 | Büchler Costa, Joana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

2

**EP 4 316 434 A1**

## ANNEX TO THE EUROPEAN SEARCH REPORT
## ON EUROPEAN PATENT APPLICATION NO.

EP 23 18 5995

This annex lists the patent family members relating to the patent documents cited in  the above-mentioned European search report.
The members are as contained in the European Patent Office EDP file on
The European Patent Office is in no way liable for these particulars which are merely given for the purpose of information.

12-09-2023

| Patent document cited in search report | | Publication date | Patent family member(s) | | Publication date |
|---|---|---|---|---|---|
| US 2008015553 | A1 | 17-01-2008 | NONE | | |
| US 4538608 | A | 03-09-1985 | CA | 1245297 A | 22-11-1988 |
| | | | EP | 0164858 A2 | 18-12-1985 |
| | | | JP | S612851 A | 08-01-1986 |
| | | | JP | H0412140 B2 | 03-03-1992 |
| | | | US | 4538608 A | 03-09-1985 |
| | | | ZA | 853101 B | 24-12-1985 |
| WO 2021155445 | A1 | 12-08-2021 | AU | 2021215707 A1 | 11-08-2022 |
| | | | CA | 3165061 A1 | 12-08-2021 |
| | | | CN | 115103657 A | 23-09-2022 |
| | | | EP | 4099967 A1 | 14-12-2022 |
| | | | IL | 294853 A | 01-09-2022 |
| | | | JP | 2023513178 A | 30-03-2023 |
| | | | US | 2023029661 A1 | 02-02-2023 |
| | | | WO | 2021155445 A1 | 12-08-2021 |
| US 2012029491 | A1 | 02-02-2012 | NONE | | |
| US 2021100688 | A1 | 08-04-2021 | NONE | | |

EPO FORM P0459

For more details about this annex : see Official Journal of the European Patent Office, No. 12/82